# EUROPEAN PATENT SPECIFICATION

(11) **EP 2 265 177 B1**
(45) Date of publication and mention of the grant of the patent: **14.03.2012**
(21) Application number: 09731465.2
(22) Date of filing: 09.04.2009
(51) Int. Cl.: A61B 5/08, A61B 5/113

(54) **MULTIPLE POLARITY PIEZOELECTRIC FILM SENSOR RESPIRATORY OUTPUT**
RESPIRATORISCHER AUSGANG EINES PIEZOELEKTRISCHEN FOLIENSENSORS MIT MEHRFACHER POLARITÄT
CAPTEUR DE DEBIT RESPIRATOIRE A FILM PIEZOELECTRIQUE ET A POLARITES MULTIPLES

(30) Priority: 11.04.2008 US 123781 P
(43) Date of publication of application: 29.12.2010
(73) Proprietor: Dymedix Corporation, Shoreview, MN 55126 (US)
(72) Inventor: STASZ, Peter, Moundsview, MN 55112 (US)
(74) Representative: Charig, Raymond Julian
(86) International application number: PCT/US2009/002230
(87) International publication number: WO 2009/126295

(56) References cited:
- WO-A-03/022149
- US-A- 5 348 008
- US-B1- 6 702 755

## Description

### BACKGROUND

### I. Field of the Invention

This invention relates generally to an electronic signal processing circuit for adapting two polarized piezoelectric film sensor based respiratory effort belts to a conventional polysomnograph (PSG) machine of the type commonly used in sleep laboratory applications, and more particularly to an adapter that receives one polarized polyvinylidene fluoride (PVDF) film sensor signal from a chest movement respiratory effort belt and second polarized PVDF film sensor signal from an abdominal movement respiratory effort belt.

The invention creates multiple polarity indicating signal outputs from two polarized piezoelectric film sensors that are integrated into the respiratory effort belts. Therefore, a sleep disorder-diagnosing professional is being presented with a plurality of respiratory waveforms indicating whether a sleeping patient is breathing normally during sleep or indicating whether a patient is suffering from a sleep disorder.

### II. Discussion of the Prior Art

In addressing sleep related problems, such as sleep apnea, insomnia and other physiologic events or conditions occurring during sleep, various hospitals and clinics have established laboratories sometimes referred to as "Sleep Laboratories" (sleep labs). At these sleep labs, using instrumentation, a patient's sleep patterns may be monitored and recorded for later analysis so that a proper diagnosis may be made and a therapy prescribed. Varieties of sensors have been devised for providing recordable signals related to respiratory patterns during sleep. These sensors commonly are mechanical to electrical transducers that produce an electrical signal related to body movement.

For example in the Pennock U.S. Pat. No. 4,960,118, a method and apparatus for accurately measuring respiratory flow, while the subject breathes, without using a mouthpiece, face mask or any device about the head is described. The rate of change of the circumference of the rib cage (chest) and the rate of change of the circumference of the abdomen are measured using an extensible belt with series strips of polarized piezoelectric film sensors mounted thereon. The stress on the film produces an electric output proportional to the rate of application of stress when the sensor is connected to a proper electronic amplifier. Calibration is performed by measuring the circumferential changes while the subject performs an isovolume maneuver for several breaths or while the subject breathes through a pneumotachometer and mouthpiece at a variable rate for several breaths. Calibration, as described in the Pennock U.S. Pat. No. 4,960,118, is cumbersome and puts an unnecessary strain and discomfort on the patient.

In another example in the Watson U.S. Pat. No. 4,373,534 entitled "Method and Apparatus for Calibrating Respiration Monitoring System", extensive calibration is also required. The method described in the '534 patent is known in the sleep medicine industry as "Respiratory Inductance Plethysmography (RIP)". It is a well-known matter of fact within the sleep industry that RIP technology requires extensive calibration and RIP belt measurements are actually prone to polarity reversals during the sleep tests and thus have a negative impact on the official scoring of the individual sleep patients results.

In yet another example in the Watson U.S. Pat. No. 4,834, 109 entitled "Single Position Non-Invasive Calibration Technique", RIP technology calibration is at the core of the invention. It is clear to persons skilled in the art that calibration is a key factor in the application and success of RIP technology.

US 5348008 describes a cardiorespiratory alert system that uses chest expansion transducers on a chest band and an abdominal band to sense respiration and to distinguish between normal mechanism of breathing (chest and abdomen moving together in phase) and obstructive or paradoxical breathing (chest and abdomen moving out of phase). The system includes signal processing circuitry to extract a low-frequency signal for respiration and a highfrequency one for pulse. US 6702755 describes an adaptor for interfacing a pyro/piezo sensor to a polysomnograph machine.

### SUMMARY

The present inventor has recognized, among other things, that there is a need to provide an apparatus and method that does not require the patient to go through an extensive calibration procedure as described in the Pennock U.S. Pat. No. 4,960,118, Watson U.S. Pat. No 4,373,534 and Watson U.S. Pat. No 4,834,109.

An apparatus and method that uses two independent polarized piezoelectric film sensors in order to provide a rigid phase and polarity relationship between respiratory effort movement (inhaling and exhaling) to final graphical indication of the individually processed polarized piezoelectric film sensor signals on the PSG machine display is needed.

Furthermore, there is also a need to provide an apparatus and method capable of using two independent polarized piezoelectric film sensors in order to provide a rigid phase relationship between respiratory effort movement (inhaling and exhaling) to final graphical indication of the summed polarized piezoelectric film sensor signals on the PSG machine display.

The polarized piezoelectric film sensor based respiratory effort belts of the present invention can contain PVDF film sensors, which act like a precharged polarized capacitor that provides changes in capacitive reactance (impedance) during breathing and non-breathing events. In comparison, Respiratory Inductance Plethysmography (RIP) belts consists of an array of specially arranged wires that must be locally excited by a low current, high frequency external electrical oscillator circuit. Although small, this external electrical oscillator unit is subject to wear and tear, signal loss, and frequent replacement, at significant cost, not to mention replacement of the expensive RIP belts.

To successfully market, these new types of polarized piezoelectric film sensors along with electronic sensor signal adapters, it is desirable that they not require extensive calibration and do not provide false respiratory effort results during sleep tests while still being able to be used with existing polysomnograph machines already in place in sleep laboratories.

Certain embodiments of the present invention provide an adaptor for interfacing two polarized piezoelectric film sensors to a PSG machine. The adapter comprises two independent sets of differential amplifier and integrator circuits with resistive reset having a pair of input terminals that are adapted to be coupled to the polarized piezoelectric film sensor and output terminal. The differential amplifier and signal integrator with resistive reset are configured to provide a predetermined gain factor by which the polarized piezoelectric film sensor output signal is amplified, to provide input signal averaging over time to reduce unwanted differential noise, and to significantly attenuate common-mode noise. Each of the outputs of the two differential amplifier and signal integrator with resistive reset circuits is fed to a set of signal output attenuators and to a summing node and to two stage inverting signal integrators with resistive reset.

By utilizing a differential input amplifier with a predetermined gain factor and by appropriately conditioning the amplified polarized piezoelectric film sensor output signal, the resulting three different filters can be readily matched to existing PSG electronic head boxes already on hand in most sleep laboratories.

A non-obvious aspect of the invention is that the adapter itself requires no calibration. There are no adjustable components as part of this assembly that might require tuning during a calibration procedure.

In certain examples, the integrator with resistive reset circuit can be crucial for the application when the two polarized piezoelectric film sensors are part of a respiratory effort belt system because PVDF film senses mechanical motion/stresses well into the GHz range. Because the normal human adult at rest respiration rate is relatively slow (12 to 20 breaths per minute), irregular in frequency and amplitude and of distorted sinusoidal form, the raw polarized piezoelectric film sensor signals must be averaged over time in order to condition the signal for graphical presentation in the PSG machine. Sensor signal averaging over time removes undesired patient motion signals and other environmentally induced noise signal artifacts.

The two Belt Sensors comprising the present invention are applied to a patient during sleep study recordings. The Belt Sensor provides a small voltage signal in relationship to a mechanical change due to breathing. When the chest belt sensor is being stretched, then the patient is inhaling. The signal is provided to the user's external sleep recording device. Trained medical professionals examine the recording to provide an assessment of breathing by chest and abdominal cavity movements during sleep.

Polarized lead wires are provided to interface between the belt Sensors and the user recorder. One wire may be outfitted with a red marking and is designated positive. The other wire may be outfitted with a black marking and is designated negative. The PVDF film generates a direct current (DC) voltage, much like a battery, when subjected to a mechanical stress such as stretching during inhaling. Inhaling means that the PVDF film is being stretched. Exhaling results in that the PVDF film is becoming relaxed. Lead Wires are provided to interface between the Sensor and user recorder. The positive designated PVDF film surface electrode becomes negatively charged when exhaling. The positive designated PVDF film surface electrode becomes positively charged when inhaling. The negative designated PVDF film surface electrode becomes negatively charged when inhaling. The negative designated PVDF film surface electrode becomes positively charged when exhaling. The PSG display is indicated by an upward deflection when inhaling.

When a negative voltage/charge is presented to the PSG input reference terminal, an upward deflection on the PSG display indicates a respiratory exhalation effort. When a positive voltage/charge is presented to the PSG input reference terminal, a downward deflection on the PSG display indicates a respiratory inhalation effort.

In order to maintain the polarity processing properties and to minimize potentially long phase delays as part of the invention, all electronic signal-processing paths are DC coupled. Persons skilled in the art will recognize the DC coupling when reviewing the disclosed schematic diagram of the invention by the absence of capacitors in the forward signal paths in any of the electronic building blocks.

Further areas of applicability of the present invention will become apparent from the description provided herein. It should be understood that the description and specific examples are intended for purposes of illustration only and are not intended to limit the scope of the present disclosure.

In an example, multiple polarity indicating outputs can be created using two polarized piezoelectric film sensors. Typically, the two polarized piezoelectric film sensors are constructed of polyvinylidene (PVDF) film and are part of a set of respiratory effort sensing belts. The apparatus contains a chest signal-processing channel and an abdominal signal-processing channel. The input section of the chest signal-processing channel consists of a differential amplifier and integrator with resistive reset coupled to receive the polarized piezoelectric film signal from the PVDF film transducer that makes up the sensing apparatus in the chest effort-sensing belt. The input section of the abdominal signal-processing channel consists of a differential amplifier and integrator with resistive reset coupled to receive the polarized piezoelectric film sensor signal from the PVDF film transducer that makes up the sensing apparatus in the abdominal effort-sensing belt. Both chest and abdominal differential amplifiers and integrators with resistive reset provide load impedance, voltage gain and signal averaging over time while rejecting differential and common mode noise. The output of the chest differential amplifier and integrator with resistive reset connects to the input of a third order Butterworth low-pass filter for further signal shaping and conditioning. The output of the abdominal differential amplifier and integrator with resistive reset connects to the input of a third order Butterworth low-pass filter for further signal shaping and conditioning. The outputs of the chest signal third order Butterworth low pass filter and the output of the abdomen signal third order Butterworth low pass filter are added in a summing node of a sum channel two-stage signal integrator with resistive reset. The shaped output of the sum signal two-stage integrator with resistive reset passes through the sum signal output attenuator for conditioning so that the sleep therapy professional recognizes the resulting summed chest and abdomen movement on the polysomnograph machine (PSG) more commonly. In addition, each shaped output of the chest and abdomen third order Butterworth low pass filters pass through separate chest and abdominal signal attenuators for conditioning so that the specific PSG machine displays the resulting waveforms optimally.

### DESCRIPTION OF THE DRAWINGS

The drawings described herein are for illustration purposes only and are not intended to limit the scope of the present disclosure in any way.

The forgoing features, objects and advantages of the invention will become apparent to those skilled in the art from the following detailed description of a preferred embodiment, especially when considered in conjunction with the accompanying drawings in which like the numerals in the several views refer to the corresponding parts:
FIG. 1 is a general block diagram of the adapter module comprising a preferred embodiment of the present invention;
FIG. 2 is a more detailed block diagram of the adapter module comprising a preferred embodiment of the present invention; and
FIG. 3 is a schematic diagram of the adapter module comprising a preferred embodiment showing a detailed implementation thereof.

### DESCRIPTION OF THE PREFERRED EMBODIMENT

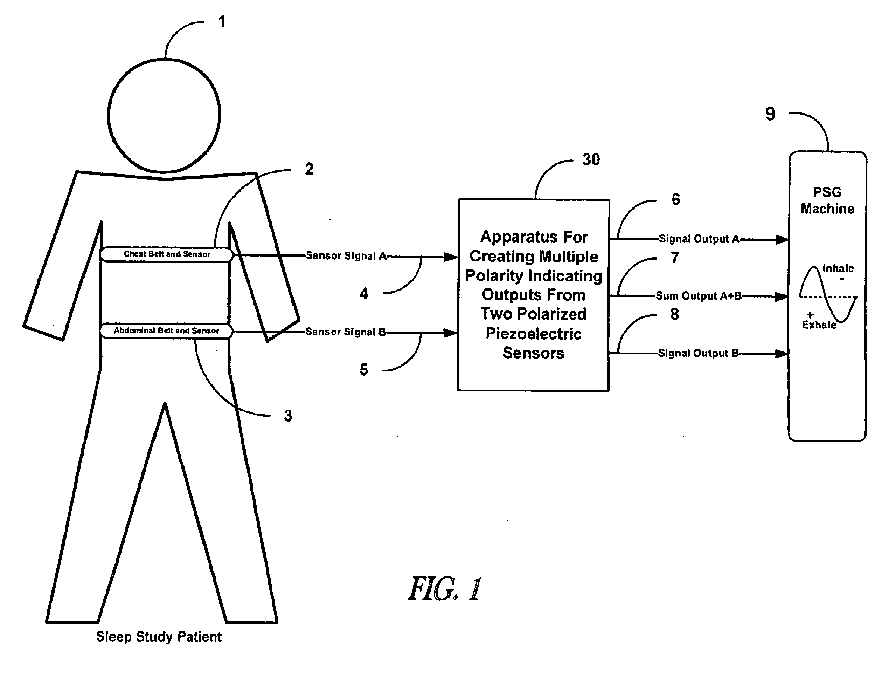

The present invention can be readily understood from Figures 1 through 3 and the following description.

The following description is merely exemplary in nature and is not intended to limit the present disclosure, application, or uses. It should be understood that throughout the drawings, corresponding reference numerals indicate like or corresponding parts and features.

A typical overall use and configuration of the adapter module is shown with the aid of FIG. 1. Referring to FIG. 1, there is indicated generally by numeral **1** a typical sleep laboratory patient who has been outfitted with one chest belt **2** and one abdominal belt **3** to measure respiratory effort. Each belt includes an integral polarized piezoelectric film sensor as may be found in the Stasz invention "Respiratory Sensing Effort Belt Using Piezo Film", Serial No. 11/743839, filed May 3, 2007, the teachings of which are hereby incorporated by reference. A pair of chest belt sensor output wire leads **4** and a pair of abdominal belt sensor output wire leads **5** connect the sleep laboratory patient **1** to the adapter apparatus for creating multiple polarity indicating outputs from two polarized piezoelectric film sensors **30.**

This specific exemplary of the embodiment shows three polarity indicating output wire pairs **6, 7** and **8** connecting the apparatus for creating multiple polarity indicating outputs from two polarized piezoelectric film sensors to a conventional, commercially-available PSG machine **9,** such as a Model Sandman available from Covidien of Kanata, ON Canada Model Alice available from Respironics Inc of Murrysville, PA Model Connex available from Natus Medical of Oakville, ON Canada Model Harmonie-S available from Stellate of Montreal, QC Canada Model Polysmith available from Nihon Kohden America of Foothill Ranch, CA Model Comet available from Astro-Med, Inc of West Warwick, RI Model Embletta available from Embla of Broomfield, CO Model E Series available from Compumedics of Charlotte, NC Model 20B available from CleveMed of Cleveland, OH Model Somnostar available from Cardinal Health of Yorba Linda, CA Model Easy II available from Cadwell Laboratories, Inc of Kennewick, WA Model Pursuit Sleep2 available from Braebon of Ogdensburg, NY Model SleepScan available from Natus Medical of Mundelein, IL All trademarks are property of their respective owners! This list is only exemplary in nature and does not claim to be comprehensive or complete.

In a typical sleep laboratory application, signal output A **6** is configured to produce the chest movement showing inhalation as an upward deflection of respiratory effort and showing exhalation as a downward deflection of respiratory effort on the PSG machine **9** display.

Furthermore, sum signal output A+B 7 is configured to produce the sum of the phase and amplitude for the chest movement sensor signal and the abdominal movement sensor signal as an upward deflection of respiratory effort during inhalation and showing exhalation as a downward deflection of respiratory effort on the PSG machine **9** display.

Furthermore, signal output B **8** is configured to produce the abdominal movement signal from the piezoelectric film sensor associated with the abdominal belt 3 showing inhalation as an upward deflection of respiratory effort and showing exhalation as a downward deflection of respiratory effort on the PSG machine **9** display.

It is by international convention and by requirement of the American Association of Sleep Medicine (AASM) that a patient's inhalation produces an upward deflection and exhalation produces a downward deflection on the PSG machine **9** display.

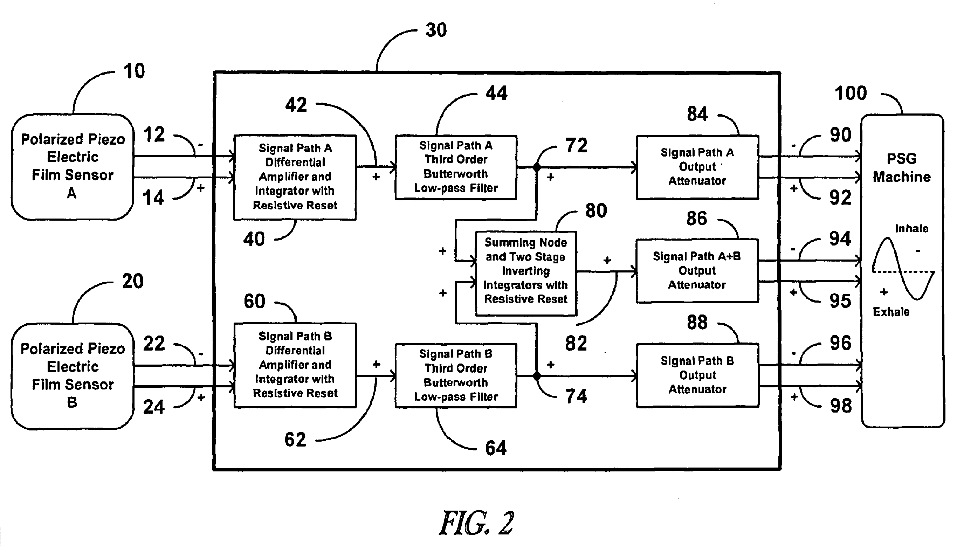

Referring next to FIG. 2, there is indicated generally by numeral 30 the functional components comprising the adapter module of the present invention.

The polarized piezoelectric film sensor A **10** in general and more specifically, the chest belt sensor, is preferably constructed in accordance with the teachings of the aforereferenced patent application Serial No. 11/743839 of Peter Stasz and entitled "Respiratory Sensing Belt Using Piezo Film". The sensor **10** is adapted to be placed on a subject's chest so that inspiratory and expiratory chest cavity movements create mechanical stress on the sensor as the belt 2 stretches and contracts.

The polarized piezoelectric film sensor A **10** connects to the signal path A differential amplifier and integrator with resistive reset **40** via a pair of input wire leads **12-14.**

Wire **12** of the input wire pair is indicated to represent the positive terminal of the polarized piezoelectric film sensor that goes positive when patient is inhaling.

Wire **14** of the input wire pair is indicated to represent the negative terminal of the polarized piezoelectric film sensor that goes positive when patient is inhaling. The differential amplifier and integrator with resistive reset **40** of the signal path A comprises a differential type amplifier which functions to increase the common-mode rejection of the adapter system so as to make it less susceptible to 60Hz noise present in the environment as well as to motion artifacts. The signal integrator with resistive reset serves to slowly average the incoming signal over time so that the differential amplifier only amplifies signals that are within the response time of interest, i.e., the patient's respiratory response time. The averaging signal integrator may operate with a fixed time constant of about 62.5 ms. This value has been selected and found to be working optimally during operation and performance regarding respiratory effort.

Without limitation, the differential amplifier and integrator with resistive reset **40** may have a gain in the range of from 2 to 10 with about 6.2 being quite adequate.

The output signal **42** from the differential input amplifier and integrator with resistive reset **40** is applied to a third order Butterworth low pass filter **44.** The input of the third order Butterworth filter **44** is connected to the output, terminal **42** of the differential input amplifier **40.**

It should be understood by those skilled in the art that the type of filter response is neither limited to a third order filter nor is it limited to a Butterworth response. Other filter responses may also be used.

Typically, but not limited to, the cut-off frequency for the third order Butterworth low pass filter **44** may be about 500mHz.

The output **72** of the signal path A third order Butterworth low-pass filter module **44** connects to the input of the signal path A output attenuator module **84.**

The output attenuator for signal path A **84** attenuates the signal coming from the signal path A third order Butterworth low-pass filter **44** in order to reduce the signal path A amplitude to a level that is compliant with the requirements of the input specifications of the input jack of the PSG machine **100** by way of lines **90** and **92** respectively.

It should be clear to those skilled in the art that the entire signal path A starting from the polarized piezoelectric film sensor **10** and ending at the PSG machine **100** is DC coupled, thus ensuring that the relationship of polarized piezoelectric film sensor polarity and indication of respiration effort between inhalation and exhalation on the PSG machine is purposely maintained.

The polarized piezoelectric film sensor B **20** in general and more specifically, the chest belt sensor, is also preferably constructed in accordance with the teachings of the aforereferenced Peter Stasz application entitled "Respiratory Sensing Belt Using Piezo Film". The sensor B **20** is adapted to be placed on a subject's abdomen so that inspiratory and expiratory belly movements create mechanical stress on the sensor.

The polarized piezoelectric film sensor B **10** connects to the signal path B differential amplifier and integrator with reset **60** via a pair of input wire leads **22-24.**

Wire **22** of the input wire pair is indicated to represent the positive terminal of the polarized piezoelectric film sensor that goes positive when patient is inhaling.

Wire **24** of the input wire pair is indicated to represent the negative terminal of the polarized piezoelectric film sensor that goes positive when patient is inhaling. The differential amplifier and integrator with resistive reset **60** of the signal path A comprises a differential type amplifier which functions to increase the common-mode rejection of the adapter system so as to make it less susceptible to 60Hz noise present in the environment as well as to motion artifacts. The signal integrator with resistive reset functions to slowly average the incoming signal over time so that the differential amplifier only amplifies signals that are within the response time of interest, more specifically the patient's respiratory response time. The averaging signal integrator preferably operates with a fixed time constant of about 62.5ms. This value has been selected and found to be working optimally during operation and performance regarding respiratory effort.

Without limitation, the differential amplifier and integrator with resistive reset **60** may have a gain in the range of from 2 to 10 with about 6.2 being quite adequate.

The output signal **62** from the differential input amplifier and integrator with resistive reset **60** is also applied to a third order Butterworth low pass filter ***64.** The input of the third order Butterworth filter **64** is connected to the output terminal **62** of the differential input amplifier and integrator with resistive reset **60.**

It is to be understood by those skilled in the art that the type of filter response is neither limited to a third order filter nor is it limited to a Butterworth response. Other filter responses may be used.

Typically, the cut-off frequency for the third order Butterworth low pass filter **64** may be about 500mHz.

The output **74** of the signal path B third order Butterworth low-pass filter **64** connects to the input of the signal path B output attenuator **88.**

The output attenuator for signal path B **88** attenuates the signal coming from the signal path B third order Butterworth low-pass filter **64** in order to reduce the signal path B amplitude to a level that is compliant with the requirements of the input specifications of the input jack of the PSG machine **100** by way of lines **96** and **98** respectively.

It should be clear to those skilled in the art that the entire signal path B starting from the polarized piezoelectric film sensor **20** and ending at the PSG machine **100,** is DC coupled. This ensures that the relationship of polarized piezoelectric sensor film polarity and indication of respiration effort between inhalation and exhalation on the PSG machine is purposely maintained.

In order to create polarity indicating signal path A (chest belt sensor) and signal path **B** (abdominal belt sensor) sum output for connection and presentation to the PSG machine **100** display, the output **72** of the signal path A third order Butterworth low-pass filter **44** and the output **74** of the signal path B third order Butterworth low-pass filter **64** are added in the summing node of a two-stage inverting integrators with resistive reset **80.**

Summing node and two stage inverting integrators with resistive reset **80** slowly average the summed signal path A and signal path B (chest and abdomen) output signals **72** plus **74** over time so that the signals that are outside the integrating time constant are rejected. The averaging signal integrator is preferably but not necessarily operating with a fixed time constant of about 62.5ms that has been selected and found to be working optimally during operation and performance regarding respiratory effort.

The output line **82** of the summing node and two stage-inverting integrators with resistive reset **80** connects to the input of the signal path A+B output attenuator **86.**

The output attenuator for signal path A+B **86** attenuates the signal coming from the summing node and two stage inverting integrators with resistive reset **80** in order to reduce the signal path A+B amplitude to a level that is compliant with the requirements of the input specifications of the input jack of the PSG machine **100** by way of lines **94** and **95** respectively.

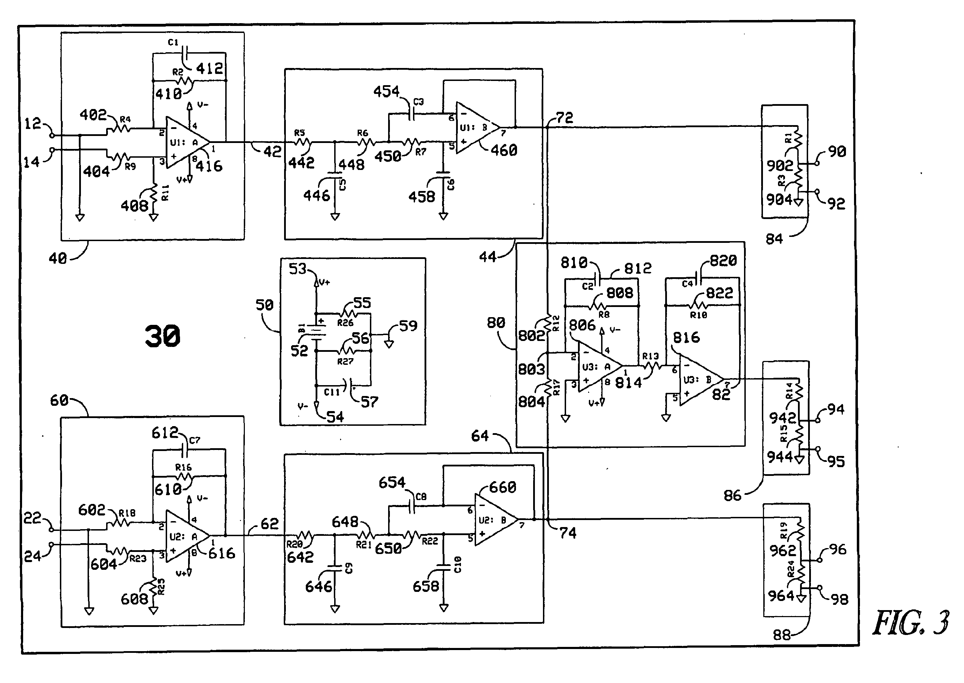

Having described the overall configuration of the adapter module with the aid of FIG. 2, a more detailed explanation of a specific implementation of the adapter will now be presented and, in that regard, reference is made to the schematic diagram of FIG. 3. Figure 3 describes in detail the building blocks outlined in Figure 2.

The adapter 30 of the present invention is integral with the cable used to couple the two polarized piezoelectric film (chest and abdominal) sensors **10** and **20** respectively to the polysomnograph machine. As such, it incorporates its own power supply and virtual ground generator **50** in the form of a single lithium battery **52** with its positive battery voltage terminal **53** identified as v+ and its negative battery voltage terminal **54** labeled v- The resistor **55** connects the positive battery voltage terminal to the virtual ground point **59.** The resistor **56** connects the negative battery voltage terminal to the virtual ground point **59.** Resistors **55** and **56** are equal in value in establishing virtual ground point **59.** The polarized capacitor **57** connects in parallel with resistors **56** to form a low alternating current (AC) impedance return path from the negative battery terminal **54** to the virtual ground point **59.**

The input terminal **12** to the differential amplifier and integrator with resistive reset **40** is coupled, via resistor **402** to the inverting input of operational amplifier **416,** to the gain setting and integrator resetting resistor **410** and to the integrating capacitor **412.** The input terminal **14** connects to the non-inverting input of differential operational amplifier and integrator with resistive reset **116,** via resistor **404** and to the input load resistor **408.**

The output from the differential input amplifier circuit **416** appears at junction **42** and connects to the signal path A third order Butterworth low-pass filter circuit **44.**

Referring to filter circuit **44,** the input appearing at junction **42** is applied, via series connected resistors **442, 448** and **450,** to the non-inverting input of an operational amplifier **460** and those resistors, along with capacitors **446, 454** and **458** cooperate with the operational amplifier **460** to function as a low-pass filter. The output of the operational amplifier **460** is presented to node **72.**

The values of the resistors **442, 448** and **450** and the capacitors **446, 454** and **458** may be set to establish a cut-off frequency of the third order Butterworth low-pass filter circuit **44** to about 500mHz as mentioned previously.

Node **72** feeds into the signal path A output attenuator **84.**

The signal path A output attenuator **84** consists of a voltage divider including resistors **902** and **904** to drop the polarized piezoelectric film sensor based signal component to acceptable levels of the PSG machine to which the polarized piezoelectric film sensor is being interfaced via a pair of lead wires **90** and **92** respectively.

The input terminal **22** to the differential amplifier and integrator with resistive reset **60** is coupled, via resistor **602** to the inverting input of operational amplifier **616,** to the gain setting and integrator-resetting resistor **610** and to the integrating capacitor **612.** The input terminal **24** to the differential amplifier and integrator with resistive reset **60** is coupled, via resistor **604** to the non-inverting input of operational amplifier **616** and to the input load resistor **608.**

The output from the differential input amplifier circuit **616** appears at junction **62** and connects to the signal path B third order Butterworth low-pass filter circuit **64.**

Referring to filter circuit **64,** the input appearing at junction **62** is applied, via series connected resistors **642, 654** and **650,** to the non-inverting input of an operational amplifier **660** and those resistors, along with capacitors **646, 652** and **658** cooperate with the operational amplifier **660** to function as a low-pass filter. The output of the operational amplifier **660** is presented to node **74.**

The values of the resistors **642, 648** and **650** and the capacitors **646, 654** and **658** may be set to establish a cut-off frequency of the third order Butterworth low-pass filter circuit **64** to about 500mHz as mentioned previously.

Node **74** feeds into the signal path B output attenuator **88.**

The signal path B output attenuator **88** consists of a voltage divider including resistors **962** and **964** to drop the polarized piezoelectric film sensor based signal component to acceptable levels of the PSG machine to which the polarized piezoelectric film sensor is being interfaced via a pair of lead wires **96** and **98** respectively.

Signal nodes **72** and **74** feed, via resistors **802** and **804** respectively into the signal A and signal B summing node **803** of circuit **80.** The inverting input of operational amplifier **806** is also the first stage of the inverting integrating integrator with resistive reset circuit **80.** The non-inverting input of the operational amplifier **806** connects to virtual ground **59.**

Resistor **808** sets the first stage amplifier gain to unity and resets the integrating capacitor **810** which it is connected to in parallel. The integrating capacitor **810** is connected on one side to the summing node **803** and the inverting input of the operational amplifier **806.** The other side of the integrating capacitor **810** is connected to the operational amplifier output node **812.** Resistor **808** and capacitor **810** set up the averaging RC (resistance times capacitance) time constant for the first integrator stage. The first stage averaging signal integrator **806** is operating with a fixed time constant of around 62.5ms that has been selected and found to be working optimally during operation and performance regarding respiratory effort.

The output of the first inverting integrator with resistive reset **812** feeds into the inverting input terminal of the second inverting integrator with resistive reset stage via input resistor **814.**

The inverting input of operational amplifier **816** is also the second stage of the inverting integrating integrator with resistive reset **80.** The non-inverting input of the operational amplifier **816** connects to virtual ground **59.**

Resistor **822** sets the second stage amplifier gain to unity and resets the integrating capacitor **820** which it is connected to in parallel. The integrating capacitor **820** is connected on one side to the input resistor **814** and the inverting input of the operational amplifier **816.** The other side of the integrating capacitor **820** is connected to the operational amplifier output node **82.** Resistor **822** and capacitor **820** set up averaging RC time constant for the second integrator stage. The second stage averaging signal integrator is preferably also operating with a fixed time constant of about 62.5ms that has been selected and found to be working optimally during operation and performance regarding respiratory effort.

Node **82** feeds into the signal path A+B output attenuator **86.**

The signal path A+B output attenuator **86** consists of a voltage divider including resistors **942** and **944** to drop the polarized piezoelectric film based signal component to acceptable levels of the PSG machine to which the polarized piezoelectric film sensor is being interfaced via a pair of lead wires **94** and **95** respectively.

One embodiment with specifically selected components of this invention was found to be operating optimally. The list of specific components used to assemble a printed circuit board assembly is known in the industry as a Bill-of-Materials (BOM). Below is the BOM for one embodiment of this invention we found to be working optimally:

| | |
|---|---|
| B1 | BR2330A/FA |
| C1 | 0.1uF |
| C2 | 0.1 uF |
| C3 | 0.1 uF |
| C4 | 0.1 uF |
| C5 | 0.39uF |
| C6 | 0.056uF |
| C7 | 0.1uF |
| C8 | 0.1uF |
| C9 | 0.39uF |
| C10 | 0.056uF |
| C11 | 10uF/tant |
| R1 | 1.00M |
| R2 | 100k |
| R3 | 100k |
| R4 | 10.0k |
| R5 | 1.00M |
| R6 | 1.00M |
| R7 | 1.00M |
| R8 | 100k |
| R9 | 1.00k |
| R10 | 100k |
| R11 | 2.70M |
| R12 | 100k |
| R13 | 100k |
| R14 | 1.00M |
| R15 | 100k |
| R16 | 100k |
| R17 | 100k |
| R18 | 10.0k |
| R19 | 1.00M |
| R20 | 1.00M |
| R21 | 1.00M |
| R22 | 1.00M |
| R23 | 1.00k |
| R24 | 100k |
| R25 | 2.70M |
| R26 | 330k |
| R27 | 330k |
| U1: A | LMC6442AIM |
| U1: B | LMC6442AIM |
| U2: A | LMC6442AIM |
| U2: B | LMC6442AIM |
| U3: A | LMC6442AIM |
| U3: B | LMC6442AIM |

During operation in a typical application, such as in a sleep laboratory, a patient is fitted with a belt-mounted polarized piezoelectric film sensor, that includes the circuit that has been described in detail here in order for sleep scientists, sleep physicians and sleep technicians to see, detect and properly diagnose specific sleep disorders and diseases which including abnormal respiratory events including events occurring in the upper airway of the patient.

This invention has been described herein in considerable detail in order to comply with the patent statutes and to provide those skilled in the art with the information needed to apply the novel principles and to construct and use such specialized components as are required. However, it is to be understood that the invention can be carried out by specifically different equipment and devices, and that various modifications, both as to the equipment and operating procedures, can be accomplished without departing from the scope of the invention itself.

The description of the various embodiments is merely exemplary in nature and, thus, variations that do not depart from the gist of the examples and detailed description herein are intended to be within the scope of the present disclosure. Such variations are not to be regarded as a departure from the scope of the present disclosure.

## Claims

1. A system, comprising:
a first respiratory effort belt (2) including a first piezoelectric film sensor (10) configured to sense a first movement indicative of respiratory effort of a subject (1);
a second respiratory effort belt (3) including a second piezoelectric film sensor (20) configured to sense a second movement indicative of respiratory effort of the subject (1);
an electronic signal processing circuit (30), coupled to the first and second piezoelectric film sensors (10, 20), the electronic signal processing circuit configured to receive first and second information from the first and second piezoelectric film sensors and to process the received first and second information to produce an electronic signal output indicative of respiratory effort of the subject;
wherein the electronic signal processing circuit (30) includes:
a first differential amplifier and signal integrator (40) with resistive reset configured to average the received first information to reduce differential noise and to attenuate common-mode noise; and
a second differential amplifier and signal integrator (60) with resistive reset configured to average the received second information to reduce differential noise and to attenuate common-mode noise ;
wherein the electronic signal output indicative of respiratory effort of the subject includes:
the averaged received first information (72);
the averaged received second information (74); and
a summation (82) of the averaged received first information and the averaged received second information; and
the system further comprising a polysomnograph machine (100), coupled to the electronic signal processing circuit (30), the polysomnograph machine configured to receive the averaged received first information (72), the averaged received second information (74), and the summation (82) of the averaged received first information and the averaged received second information from the electronic signal processing circuit (30) and to provide the averaged received first information, the averaged received second information, and the summation of the averaged received first information and the averaged received second information to a user.

2. The apparatus of claim 1, wherein the first respiratory effort belt (2) includes a chest movement respiratory effort belt and the second respiratory effort belt (3) includes an abdominal movement respiratory effort belt.

3. The apparatus of claim 2 wherein the first and second piezoelectric film sensors (10, 20) include independent polarized piezoelectric film sensors configured to provide a rigid phase and polarity relationship between respiratory effort movement.

4. The apparatus of any of claims 1 through 3, wherein the first and second differential amplifiers and signal integrators (40, 60) with resistive resets are configured to provide a predetermined gain factor by which the received first and second information is amplified, to provide signal averaging over time to reduce differential noise, and to attenuate common-mode noise.

5. The apparatus of any of claims 1 through 4, wherein the electronic signal processing circuit (30) includes:
a first third order Butterworth low pass filter (44) configured to remove components from the received first information having a frequency above approximately 500 mHz; and
a second third order Butterworth low pass filter (64) configured to remove components from the received second information having a frequency above approximately 500 mHz.

6. The apparatus of any of claims 1 through 5, wherein the first and second differential amplifiers and signal integrators (40, 60) with resistive reset are configured to average the received first and second information only during the subject's respiratory response time.

7. A method, comprising:
receiving first information indicative of respiratory effort of a subject (1) from a first piezoelectric film sensor (10) and second information indicative of respiratory effort of the subject from a second piezoelectric film sensor (20); and
processing the received first and second information to produce an electronic signal output indicative of respiratory effort of the subject, the processing including:
averaging the received first information using a first differential amplifier and signal integrator (40) with resistive reset to reduce differential noise and to attenuate common-mode noise; and separately
averaging the received second information using a second differential amplifier and signal integrator (60) with resistive reset to reduce differential noise and to attenuate common-mode noise;
wherein the producing the electronic signal output indicative of respiratory effort of the subject includes producing the averaged received first information (72), and separately, the averaged received second information (74) and a summation (82) of the averaged received first information and the averaged received second information, the method further comprising
providing the averaged received first information (72), the averaged received second information (74), and the summation (82) of the averaged received first information and the averaged received second information to a polysomnograph machine (100).

8. The method of claim 7, wherein the receiving the first and second information includes receiving first and second information from the first and second piezoelectric film sensors (10, 20) coupled to respective first and second respiratory effort belts (2, 3).

9. The method of claim 8, wherein the first respiratory effort belt (2) includes a chest movement respiratory effort belt and the second respiratory effort belt (3) includes an abdominal movement respiratory effort belt.

10. The method of any of claims 7 through 9, wherein the first and second piezoelectric film sensors (10, 20) include independent polarized piezoelectric film sensors; and
wherein the receiving the first and second information includes receiving rigid phase and polarity relationship information between respiratory effort movement from the independent polarized piezoelectric film sensors.

11. The method of any of claims 7 through 10, wherein the processing includes amplifying the received first and second information by a predetermined gain factor using the first and second differential amplifiers and signal integrators (40, 60) with resistive reset, to provide signal averaging over time to reduce differential noise, and to attenuate common-mode noise.

12. The method of any of claims 7 through 11, wherein the processing includes removing components from the received first and second information having a frequency above approximately 500 mHz using respective first and second third order Butterworth low pass filters.

13. The method of any of claims 7 through 12, wherein the averaging the received first and second information includes averaging only during the subject's respiratory response time.

## Patentansprüche

1. System mit:
einem ersten Atmungsanstrengungsgürtel (2), der einen ersten piezoelektrischen Foliensensor (10) umfasst, welcher konfiguriert ist, um eine erste Bewegung zu erfassen, die eine Atmungsanstrengung eines Subjekts (1) anzeigt;
einem zweiten Atmungsanstrengungsgürtel (3), der einen zweiten piezoelektrischen Foliensensor (20) umfasst, welcher konfiguriert ist, um eine zweite Bewegung zu erfassen, die eine Atmungsanstrengung des Subjekts (1) anzeigt;
einer elektronischen Signalverarbeitungsschaltung (30), die an den ersten und den zweiten piezoelektrischen Foliensensor (10 bzw. 20) angeschlossen ist, wobei die elektronische Signalverarbeitungsschaltung konfiguriert ist, um erste und zweite Informationen von dem ersten und zweiten piezoelektrischen Foliensensor zu empfangen und um die empfangenen ersten und zweiten Informationen zu verarbeiten, um eine elektronische Signalausgabe zu produzieren, die eine Atmungsanstrengung des Subjekts anzeigt;
wobei die elektronische Signalverarbeitungsschaltung (30) umfasst:
einen ersten Differenzverstärker und Signalintegrierer (40) mit resistivem Reset, der konfiguriert ist, um die empfangenen ersten Informationen zu mitteln, um Gegentaktrauschen zu reduzieren und Gleichtaktrauschen zu dämpfen; und
einen zweiten Differenzverstärker und Signalintegrierer (60) mit resistivem Reset, der konfiguriert ist, um die empfangenen zweiten Informationen zu mitteln, um Gegentaktrauschen zu reduzieren und Gleichtaktrauschen zu dämpfen;
wobei die elektronische Signalausgabe, die die Atmungsanstrengung des Subjekts anzeigt, umfasst:
die gemittelten empfangenen ersten Informationen (72);
die gemittelten empfangenen zweiten Informationen (74); und
eine Summe (82) der gemittelten empfangenen ersten Informationen und der gemittelten empfangenen zweiten Informationen; und
wobei das System weiterhin eine Polysomnographmaschine (100) aufweist, die an die elektronische Signalverarbeitungsschaltung (30) angeschlossen ist, wobei die Polysomnographmaschine konfiguriert ist, um die gemittelten empfangenen ersten Informationen (72), die gemittelten empfangenen zweiten Informationen (74) und die Summe (82) der gemittelten empfangenen ersten Informationen und der gemittelten empfangenen zweiten Informationen von der elektronischen Signalverarbeitungsschaltung (30) zu empfangen und um die gemittelten empfangenen ersten Informationen, die gemittelten empfangenen zweiten Informationen und die Summe der gemittelten empfangenen ersten Informationen und der gemittelten empfangenen zweiten Informationen einem Benutzer bereitzustellen.

2. Vorrichtung nach Anspruch 1, wobei der erste Atmungsanstrengungsgürtel (2) einen Brustbewegungsatmungsanstrengungsgürtel und der zweite Atmungsanstrengungsgürtel (3) einen Unterleibbewegungsatmungsanstrengungsgürtel umfasst.

3. Vorrichtung nach Anspruch 2, wobei der erste und der zweite piezoelektrische Foliensensor (10 bzw. 20) unabhängige polarisierte piezoelektrische Foliensensoren umfassen, die konfiguriert sind, um eine Starrphasen- und Polaritätsbeziehung der Atmungsanstrengungsbewegung bereitzustellen.

4. Vorrichtung nach einem der Ansprüche 1 bis 3, wobei der erste und der zweite Differenzverstärker und Signalintegrierer (40 bzw. 60) mit resistiven Resets konfiguriert sind, um einen vorgegebenen Verstärkungsfaktor bereitzustellen, mit dem die empfangenen ersten und zweiten Informationen verstärkt werden, um eine Signalmittelung über der Zeit bereitzustellen, um Gegentaktrauschen zu reduzieren und Gleichtaktrauschen zu dämpfen.

5. Vorrichtung nach einem der Ansprüche 1 bis 4, wobei die elektronische Signalverarbeitungsschaltung (30) umfasst:
einen ersten Butterworth-Lowpassfilter dritter Ordnung (44), der konfiguriert ist, um Komponenten von den empfangenen ersten Informationen zu entfernen, die eine Frequenz oberhalb ungefähr 500 mHz aufweisen; und
einen zweiten Butterworth-Lowpassfilter dritter Ordnung (64), der konfiguriert ist, um Komponenten von den empfangenen zweiten Informationen zu entfernen, die eine Frequenz oberhalb ungefähr 500 mHz aufweisen.

6. Vorrichtung nach einem der Ansprüche 1 bis 5, wobei der erste und der zweite Differenzverstärker und Signalintegrierer (40 bzw. 60) mit resistivem Reset konfiguriert sind, um die empfangenen ersten und zweiten Informationen nur während der Atmungsantwortzeit des Subjekts zu mitteln.

7. Verfahren mit:
Empfangen von ersten Informationen, die Atmungsanstrengung eines Subjekts (1) anzeigen, von einem ersten piezoelektrischen Foliensensor (10) und von zweiten Informationen, die eine Atmungsanstrengung des Subjekts anzeigen, von einem zweiten piezoelektrischen Foliensensor (20); und
Verarbeiten der empfangenen ersten und zweiten Informationen, um eine elektronische Signalausgabe zu produzieren, das Atmungsanstrengung des Subjekts anzeigt, wobei das Verarbeiten umfasst:
Mitteln der empfangenen ersten Informationen unter Verwendung eines ersten Differenzverstärkers und Signalintegrierers (40) mit resistivem Reset, um Gegentaktrauschen zu reduzieren und Gleichtaktrauschen zu dämpfen; und getrennt davon
Mitteln der empfangenen zweiten Informationen unter Verwendung eines zweiten Differenzverstärkers und Signalintegrierers (60) mit resistivem Reset, um Gegentaktrauschen zu reduzieren und Gleichtaktrauschen zu dämpfen;
wobei das Produzieren der elektronischen Signalausgabe, die Atmungsanstrengung des Subjekts anzeigt, das Produzieren der gemittelten empfangenen ersten Informationen (72) und getrennt davon der gemittelten empfangenen zweiten Informationen (74) und einer Summe (82) der gemittelten empfangenen ersten Informationen und der gemittelten empfangenen zweiten Informationen umfasst, wobei das Verfahren weiterhin aufweist
Bereitstellen der gemittelten empfangenen ersten Informationen (72), der gemittelten empfangenen zweiten Informationen (74) und der Summe (82) der gemittelten empfangenen ersten Informationen und der gemittelten empfangenen zweiten Informationen an eine Polysomnographmaschine (100).

8. Verfahren nach Anspruch 7, wobei das Empfangen der ersten und zweiten Informationen das Empfangen der ersten und zweiten Informationen von dem ersten und zweiten piezoelektrischen Foliensensor (10 bzw. 20) angeschlossen an den jeweiligen ersten und zweiten Atmungsanstrengungsgürtel (2 bzw. 3) umfasst.

9. Verfahren nach Anspruch 8, wobei der erste Atmungsanstrengungsgürtel (2) einen Brustbewegungsatmungsanstrengungsgürtel und der zweite Atmungsanstrengungsgürtel (3) einen Unterleibsbewegungsatmungsanstrengungsgürtel umfasst.

10. Verfahren nach einem der Ansprüche 7 bis 9, wobei der erste und der zweite piezoelektrische Foliensensor (10 bzw. 20) unabhängige polarisierte piezoelektrische Foliensensoren umfassen; und
wobei das Empfangen der ersten und zweiten Informationen das Empfangen von Starrphasen- und Polarisationsbeziehungsinformationen der Atmungsanstrengungsbewegung von den unabhängigen polarisierten piezoelektrischen Foliensensoren umfasst.

11. Verfahren nach einem der Ansprüche 7 bis 10, wobei das Verarbeiten das Verstärken der empfangenen ersten und zweiten Informationen um einen vorgegebenen Verstärkungsfaktor unter Verwendung des ersten und des zweiten Differenzverstärkers und Signalintegrierers (40 bzw. 60) mit resistivem Reset umfasst, um Signalmittelung über der Zeit bereitzustellen, um Gegentaktrauschen zu reduzieren und um Gleichtaktrauschen zu dämpfen.

12. Verfahren nach einem der Ansprüche 7 bis 11, wobei das Verarbeiten das Entfernen von Komponenten von den empfangenen ersten und zweiten Informationen mit einer Frequenz oberhalb von ungefähr 500 mHz unter Verwendung jeweiliger erster und zweiter Butterworth-Lowpassfilter dritter Ordnung umfasst.

13. Verfahren nach einem der Ansprüche 7 bis 12, wobei das Mitteln der empfangenen ersten und zweiten Informationen das Mitteln nur während der Atmungsantwortzeit des Subjekts umfasst.

## Revendications

1. Système comprenant :
une première ceinture d'effort respiratoire (2) comprenant un premier capteur à film piézoélectrique (10) configuré pour capter un premier mouvement indicatif de l'effort respiratoire d'un sujet (1) ;
une seconde ceinture d'effort respiratoire (3) comprenant un second capteur à film piézoélectrique (20) configuré pour détecter un second mouvement indicatif de l'effort respiratoire du sujet (1) ;
un circuit de traitement de signal électronique (30), couplé aux premier et second capteurs de film piézoélectrique (10, 20), le circuit de traitement de signal électronique étant configuré pour recevoir des première et seconde informations des premier et second capteurs à film piézoélectrique et pour traiter les première et seconde informations reçues afin de produire une sortie de signal électronique indicative de l'effort respiratoire du sujet ;
dans lequel le circuit de traitement de signal électronique (30) comprend :
un premier amplificateur différentiel et intégrateur de signal (40) avec réinitialisation résistive configuré pour établir la moyenne de la première information reçue afin de réduire le bruit différentiel et d'atténuer le bruit de mode commun ; et
un second amplificateur différentiel et intégrateur de signal (60) avec réinitialisation résistive configuré pour établir la moyenne de la seconde information reçue afin de réduire le bruit différentiel et d'atténuer le bruit de mode commun ;
dans lequel la sortie de signal électronique indicative d'un effort respiratoire du sujet comprend :
la première information reçue moyennée (72) ;
la seconde information reçue moyennée (74) ; et
une sommation (82) de la première information reçue moyennée et de la seconde information reçue moyennée ; et
le système comprend en outre une machine polysomnographe (100), couplée au circuit de traitement de signal électronique (30), la machine polysomnographe étant configurée pour recevoir la première information reçue moyennée (72), la seconde information reçue moyennée (74), et la sommation (82) de la première information reçue moyennée et de la seconde information reçue moyennée provenant du circuit de traitement de signal électronique (30) et pour fournir la première information reçue moyennée, la seconde information reçue moyennée, et la sommation de la première information reçue moyennée et de la seconde information reçue moyennée à un utilisateur.

2. Appareil selon la revendication 1, dans lequel la première ceinture d'effort respiratoire (2) comprend une ceinture d'effort respiratoire de mouvement de poitrine et la seconde ceinture d'effort respiratoire (3) comprend une ceinture d'effort respiratoire de mouvement abdominal.

3. Appareil selon la revendication 2, dans lequel les premier et second capteurs à film piézoélectrique (10, 20) comprennent des capteurs à film piézoélectrique polarisé indépendants configurés pour fournir une relation rigide de phase et de polarité entre un mouvement d'effort respiratoire.

4. Appareil selon l'une quelconque des revendications 1 à 3, dans lequel les premier et second amplificateurs différentiels et intégrateurs de signal (40, 60) avec réinitialisations résistives sont configurés pour fournir un facteur de gain prédéterminé par lequel les première et seconde informations reçues sont amplifiées, pour fournir une moyenne de signal au cours du temps afin de réduire le bruit différentiel, et d'atténuer le bruit de mode commun.

5. Appareil selon l'une quelconque des revendications 1 à 4, dans lequel le circuit de traitement de signal électronique (30) comprend :
un premier filtre passe-bas Butterworth de troisième ordre (44) configuré pour éliminer des composantes de la première information reçue ayant une fréquence supérieure à approximativement 500 mHz ; et
un second filtre passe-bas Butterworth de troisième ordre (64) configuré pour éliminer des composantes de la seconde information reçue ayant une fréquence supérieure à approximativement 500 mHz.

6. Appareil selon l'une quelconque des revendications 1 à 5, dans lequel les premier et second amplificateurs différentiels et intégrateurs de signal (40, 60) avec réinitialisations résistives sont configurés pour établir la moyenne des première et seconde informations reçues uniquement pendant le temps de réponse respiratoire du sujet.

7. Procédé, comprenant :
la réception d'une première information indicative de l'effort respiratoire d'un sujet (1) à partir d'un premier capteur à film piézoélectrique (10) et d'une seconde information indicative de l'effort respiratoire du sujet à partir d'un second capteur à film piézoélectrique (20) ; et
le traitement des première et seconde informations reçues afin de produire une sortie de signal électronique indicative de l'effort respiratoire du sujet, le traitement comprenant :
l'établissement de la moyenne de la première information reçue à l'aide d'un premier amplificateur différentiel et intégrateur de signal (40) avec réinitialisation résistive afin de réduire le bruit différentiel et d'atténuer le bruit de mode commun ; et séparément
l'établissement de la moyenne de la seconde information reçue à l'aide d'un second amplificateur différentiel et intégrateur de signal (60) avec réinitialisation résistive afin de réduire le bruit différentiel et d'atténuer le bruit de mode commun ;
dans lequel la production de la sortie de signal électronique indicative de l'effort respiratoire du sujet comprend la production de la première information reçue moyennée (72), et séparément, de la seconde information reçue moyennée (74) et d'une sommation (82) de la première information reçue moyennée et de la seconde information reçue moyennée, le procédé comprenant en outre :
la fourniture de la première information reçue moyennée (72), de la seconde information reçue moyennée (74), et de la sommation (82) de la première information reçue moyennée et de la seconde information reçue moyennée à une machine polysomnographe (100).

8. Procédé selon la revendication 7, dans lequel la réception des première et seconde informations comprend la réception des première et seconde informations des premier et second capteurs à film piézoélectrique (10, 20) couplés aux première et seconde ceintures d'effort respiratoire (2, 3) respectives.

9. Procédé selon la revendication 8, dans lequel la première ceinture d'effort respiratoire (2) comprend une ceinture d'effort respiratoire de mouvement de poitrine et la seconde ceinture d'effort respiratoire (3) comprend une ceinture d'effort respiratoire de mouvement abdominal.

10. Procédé selon l'une quelconque des revendications 7 à 9, dans lequel les premier et second capteurs à film piézoélectrique (10, 20) comprennent des capteurs à film piézoélectrique polarisés indépendants ; et
dans lequel la réception des première et seconde informations comprend la réception d'information de relation rigide de phase et de polarité entre un mouvement d'effort respiratoire provenant des capteurs à film piézoélectrique polarisés indépendants.

11. Procédé selon l'une quelconque des revendications 7 à 10, dans lequel le traitement comprend l'amplification des première et seconde informations reçues par un facteur de gain prédéterminé à l'aide des premier et second amplificateurs différentiels et intégrateurs de signal (40, 60) avec réinitialisation résistive, afin de produire une moyenne de signal au cours du temps pour réduire le bruit différentiel et pour atténuer le bruit de mode commun.

12. Procédé selon l'une quelconque des revendications 7 à 11, dans lequel le traitement comprend l'élimination des composantes des première et seconde informations reçues ayant une fréquence supérieure à approximativement 500 mHz à l'aide des premier et second filtres passe-bas Butterworth de troisième ordre respectifs.

13. Procédé selon l'une quelconque des revendications 7 à 12, dans lequel l'établissement de la moyenne des première et seconde informations reçues comprend l'établissement de la moyenne uniquement pendant le temps de réponse respiratoire du sujet.
